# EUROPÄISCHE PATENTSCHRIFT

(11) **EP 3 332 730 B1**
(45) Veröffentlichungstag und Bekanntmachung des Hinweises auf die Patenterteilung: **03.11.2021**
(21) Anmeldenummer: 17185328.6
(22) Anmeldetag: 08.08.2017
(51) Int. Cl.: A61B 90/98, A61B 90/00, A61B 34/20

(54) **VERFAHREN UND TRACKINGSYSTEM ZUM NACHVERFOLGEN EINES MEDIZINISCHEN OBJEKTS**
METHOD AND TRACKING SYSTEM FOR TRACKING A MEDICAL OBJECT
PROCÉDÉ ET SYSTÈME DE SUIVI PERMETTANT LE SUIVI D'UN OBJET MÉDICAL

(43) Veröffentlichungstag der Anmeldung: 13.06.2018
(73) Patentinhaber: Siemens Healthcare GmbH, 91052 Erlangen (DE)
(72) Erfinder: Winneberger, David, 90439 Nürnberg (DE)

(56) Entgegenhaltungen:
- WO-A1-2012/057871
- WO-A2-2006/051523
- US-A1- 2008 300 478
- US-A1- 2010 030 061
- US-A1- 2012 083 652
- US-A1- 2014 135 744

## Beschreibung

Die Erfindung betrifft ein Verfahren und ein Trackingsystem zum automatischen Nachverfolgen eines medizinischen Objekts. Heutzutage sind medizinische Verfahren oftmals sehr komplex und können die Verwendung einer Vielzahl unterschiedlicher medizinischer Objekte, wie beispielsweise Instrumente, Werkzeuge und Implantate, umfassen. Gleichzeitig nimmt der Bedarf oder der Wunsch nach einer datengetriebenen Analyse in vielen Wirtschafts-, Technologie- und Anwendungsgebieten - so auch im Bereich medizinischer Anwendungen - stetig zu. Bisher in diesem Bereich verfügbare Methoden sind oftmals sehr zeit- und aufwandsintensiv und können, sofern sie überhaupt angewendet werden, zu einer unvollständigen und fehleranfälligen Datenerfassung führen.

Die WO 2012 / 057 871 A1 befasst sich mit einer Technik zur Fehlerkorrektur bei einer chirurgischen Navigation. Dabei werden eine Position eines Magnetfeldsensors in einem Magnetfeld und eine Beschleunigung des Magnetfeldsensors bestimmt. Eine modifizierte Position des Magnetfeldsensors wird in einem Bild eines medizinischen Patienten basierend auf der Beschleunigung und einer zuvor bestimmten Position angezeigt. Aufgabe der vorliegenden Erfindung ist es, verbesserte und besser analysierbare Abläufe in medizinischen Anwendungsbereichen zu ermöglichen.

Diese Aufgabe wird erfindungsgemäß durch die Gegenstände der unabhängigen Patentansprüche gelöst. Vorteilhafte Ausgestaltungen und Weiterbildungen der Erfindung sind in den abhängigen Patentansprüchen sowie in der Beschreibung und in den Zeichnungen angegeben. Die Durchführung des chirurgischen Verfahrens ist nicht Teil der Erfindung. Die im Folgenden dargestellten chirurgischen Schritte werden lediglich zur Veranschaulichung des bildgebenden Verfahrens erläutert und dargestellt.

Ein erfindungsgemäßes Verfahren dient zum Nachverfolgen eines medizinischen Objekts. Dabei werden mittels eines medizinischen bildgebenden Verfahrens gewonnenen Bilddaten kontinuierlich erfasst. Dies kann beispielsweise ein Aufnehmen einer Serie von Röntgen-, Angiographie- oder Tomographiebilder oder dergleichen ebenso bedeuten wie ein Auslesen entsprechender Bilddaten aus einem Datenspeicher oder eine Empfangen der Bilddaten durch eine Datenverarbeitungseinrichtung oder dergleichen. Weiterhin wird eine vorgegebene Zielposition für das medizinische Objekt erfasst. Dies kann beispielsweise ein Erfassen einer Benutzereingabe oder ein Auslesen eines Datenspeichers, in dem die Zielposition elektronisch gespeichert ist, bedeuten. Ebenso kann es möglich sein, die Zielposition automatisch zu bestimmen, beispielsweise durch Verarbeiten oder Auswerten der Bilddaten. Hierfür können auch unterstützende Daten verwendet oder berücksichtigt werden. Derartige unterstützende Daten können beispielsweise eine Art des medizinischen Objekts und/oder eine Art einer jeweiligen unter Verwendung des medizinischen Objekts durchgeführten Prozedur betreffen oder angeben. Auch diese Daten können beispielsweise durch ein Erfassen einer entsprechenden Benutzereingabe oder ein Auslesen eines entsprechenden Datenspeichers gewonnen werden.

In einem weiteren Verfahrensschritt erfolgen ein automatisches Detektieren des medizinischen Objekts sowie ein zeitaufgelöstes Nachverfolgen von dessen jeweiliger Position anhand der Bilddaten mittels eines Bildverarbeitungsalgorithmus. Mit anderen Worten werden die Bilddaten also verarbeitet beziehungsweise ausgewertet, um das medizinische Objekt in den Bilddaten zu detektieren beziehungsweise zu erkennen. Dazu befindet sich das medizinische Objekt zu einem jeweiligen Aufnahmezeitpunkt der Bilddaten selbstverständlich in einem durch diese erfassten oder abgebildeten Aufnahmebereich. Das zeitaufgelöstes Nachverfolgen der Position des medizinischen Objekts bedeutet, dass über mehrere Zeitpunkte hinweg die jeweilige Position des medizinischen Objekts zu dem jeweiligen Zeitpunkt zusammen mit dem Zeitpunkt erfasst beziehungsweise bestimmt wird. So kann als ein räumlich und zeitlich bestimmter Pfad oder eine Trajektorie des medizinischen Objekts bestimmt werden. Die jeweilige Position des medizinischen Objekts kann dabei relativ zu einem grundsätzlich beliebigen vorgegebenen Bezugspunkt in einem beliebigen vorgegebenen Koordinatensystem bestimmt werden. Bevorzugt kann die Position in einem relativ zu einem zum Aufnehmen der Bilddaten verwendeten Aufnahmegerät ortsfesten Koordinatensystem oder relativ zu einem ebenfalls mittels des bildgebenden Verfahrens beziehungsweise durch die Bilddaten zumindest teilweise erfassten oder abgebildeten Untersuchungsobjekts, beispielsweise einem Patienten, bestimmt werden.

Besonders bevorzugt kann die Position des medizinischen Objekts relativ zu einem durch die Bilddaten abgebildeten anatomischen Merkmal eines Patienten oder einer vorgegebenen Markierung oder Referenzposition bestimmt werden. Diese Referenzposition kann beispielsweise die erfasste Zielposition sein. So kann beispielsweise besonders einfach ein Abstand des medizinischen Objekts in seiner jeweils aktuellen Position zu der Zielposition bestimmt werden. Der Bildverarbeitungsalgorithmus kann beispielsweise einen Objekterkennungsschritt umfassen. Bevorzugt kann so mittels des Bildverarbeitungsalgorithmus beispielsweise das medizinische Objekt ebenso erkannt werden wie das anatomische Merkmal. Es kann besonders vorteilhaft sein als oder als Teil des Bildverarbeitungsalgorithmus beziehungsweise zu dessen Ausführung ein neuronales Netz, insbesondere ein konvolutionales neuronales Netz zu verwenden, da mittels eines entsprechend trainierten neuronalen Netzes eine besonders gute Anpassung an einen jeweiligen Anwendungsfall und somit eine besonders zuverlässige Objekterkennung ermöglicht werden kann.

In einem weiteren Verfahrensschritt wird angezeigt, dass beziehungsweise wenn das detektierte medizinische Objekt die Zielposition erreicht hat. Dass das medizinische Objekt die Zielposition erreicht hat, wird ebenfalls mittels des oder eines Bildverarbeitungsalgorithmus ermittelt. Ebenso können beispielsweise automatisch jeweilige Koordinaten der Zielposition mit jeweiligen Koordinaten der jeweils aktuellen Position des medizinischen Objekts verglichen werden. Ergibt dieser Vergleich eine unterhalb eines vorgegebenen Schwellenwertes liegende Differenz, so kann dies als Erreichen der Zielposition interpretiert werden.

In einem weiteren Verfahrensschritt werden mehrere oder alle der detektierten Positionen des medizinischen Objekts und der zugehörigen Detektionszeitpunkte in einer Datenbank gespeichert. Mit anderen Worten kann also der bestimmte räumlich und zeitlich definierte Pfad beziehungsweise die Trajektorie des medizinischen Objekts in der Datenbank gespeichert werden. Bevorzugt kann in der Datenbank auch die jeweilige Zielposition gespeichert werden. Darüber hinaus kann es besonders vorteilhaft sein, vorgegebene und/oder automatisch erkannte Ereignisse mit einem jeweiligen Eintritts- oder Erkennungszeitpunkt in der Datenbank zu speichern. Hierdurch kann dann vorteilhaft aufgrund der größeren Datenbasis eine besonders genaue und aussagekräftige Analyse eines jeweiligen Vorgangs, beispielsweise einer jeweiligen, unter Verwendung des medizinischen Objekts durchgeführten Prozedur ermöglicht werden.

Das medizinische Objekt kann beispielsweise ein Katheter, eine Gefäßstütze (Stent), eine medizinische Schraube oder dergleichen sein. Welche medizinischen Objekte beziehungsweise welche Arten von medizinischen Objekten durch die vorliegende Erfindung nachverfolgt werden können, kann im Einzelfall jeweils von dem verwendeten bildgebenden Verfahren abhängen. Es kann besonders vorteilhaft sein, mehrere medizinische Objekte in den Bilddaten zu detektieren und nachzuverfolgen. Beispielsweise kann automatisch ein in einen Patienten beziehungsweise einen mittels des bildgebenden Verfahrens abgebildeten Teilbereich des Patienten eingeführter Katheter automatisch detektiert und nachverfolgt werden. Als Zielposition kann beispielsweise ein Mittelpunkt einer Stenose markiert, also vorgegeben oder automatisch anhand der Bilddaten bestimmt werden. Im Bereich der Stenose kann bereits eine Gefäßstütze angeordnet sein, die als weiteres medizinisches Objekt ebenfalls automatisch detektiert, erkannt und nachverfolgt werden kann.

Es kann dann ein Weg oder Fahrt, also eine Bewegung des Katheters relativ zu der Stenose beziehungsweise der Zielposition nachverfolgt (getrackt) und angezeigt werden, dass beziehungsweise wenn die automatische Bildverarbeitung oder Bildauswertung ergibt, dass der Katheter die Zielposition, hier also etwa den Mittelpunkt der Stenose erreicht hat.

Beispielsweise je nach Art des medizinischen Objekts, einem jeweiligen Patientenbereich, in dem das medizinische Objekt eingesetzt wird, dem verwendeten bildgebenden Verfahren und einer erzielten Bildqualität bei einer Darstellung der Bilddaten kann eine rein auf Augenmaß beispielsweise eines behandelnden Arztes basierende präzise Verortung des medizinischen Objekts schwierig sein und/oder ein zeitaufwändiges genaues Studium der jeweiligen Darstellung erfordern. Durch die automatische Bildauswertung, welche beispielsweise eine automatische Kantendetektion umfassen kann, kann eine höhere Präzision bei der Verortung des medizinischen Objekts, insbesondere relativ zu der Zielposition und/oder relativ zu anatomischen Merkmalen oder Details, wie beispielsweise einer Gefäßwand, erzielt werden. Dabei kann zudem vorteilhaft automatisch eine jeweilige Skala oder Skalierung der Bilddaten beziehungsweise der Darstellung berücksichtigt werden, sodass beispielsweise ein entsprechender Abstand des medizinischen Objekts zu der Zielposition in absoluten metrischen Einheiten bestimmt und angegeben oder angezeigt werden kann. Hierdurch können vorteilhaft beispielsweise auf einer Nicht-Berücksichtigung eines Zoomfaktor basierende Fehlinterpretationen vermieden werden. Zudem kann so vorteilhaft eine zuverlässige Referenz bereitgestellt werden, anhand welcher sich der Arzt bei einer Handhabung des medizinischen Geräts besonders einfach, schnell und präzise orientieren kann. Insgesamt kann so die jeweilige medizinische Prozedur mit verbesserter Sicherheit, Zuverlässigkeit und Präzision durchgeführt werden, da die automatische Bildverarbeitung eine zusätzliche objektive Absicherung einer Interpretation des jeweiligen Arztes oder medizinischen Personals bietet.

Während bei herkömmlichen Verfahren die Bilddaten oftmals lediglich live während der jeweiligen Prozedur auf einem Monitor oder Bildschirm dargestellt werden, ermöglicht die vorliegende Erfindung durch das Speichern der detektierten Positionen und der entsprechenden Detektionszeitpunkte des medizinischen Objekts in der Datenbank zusätzlich ein nachträgliches Nachvollziehen und Analysieren der jeweiligen Prozedur beziehungsweise des jeweiligen Ablaufs (Workflow). Dazu kann es vorteilhaft sein, zusammen mit den Positionen und Detektionszeitpunkten auch jeweilige Einzelbilder oder Videosequenzen aus den Bilddaten in der Datenbank zu speichern, wodurch eine bessere Veranschaulichung der weiteren gespeicherten Daten und der jeweiligen Prozedur ermöglicht werden kann.

Durch das automatische Bestimmen und Abspeichern der entsprechenden Daten in der Datenbank können daraus automatisch weitere abgeleitete Daten oder Größen bestimmt beziehungsweise berechnet werden, die bei herkömmlichen Verfahren nicht zur Verfügung stehen. Dies können beispielsweise eine Momentan- oder Durchschnittsgeschwindigkeit des medizinischen Objekts und/oder ein genauer zeitlicher Abstand zwischen zwei Ereignissen sein. So kann beispielsweise eine Zeitdauer zwischen einem Eintreten des medizinischen Objekts und dem Erreichen der Zielposition automatisch bestimmt und bei Bedarf angezeigt werden. Eine derartige Zeitdauer, beispielsweise von einem Einführen des Katheters in den Patienten bis zu einem Aufblasen an der Zielposition, kann beispielsweise als Vergleichs- oder Qualitätsmetrik beziehungsweise -merkmal verwendet werden. So kann beispielsweise besonders einfach und objektiv eine Effizienz der jeweiligen Prozedur und/oder des jeweiligen beteiligten medizinischen Personals bestimmt beziehungsweise als Vergleichsgröße verwendet werden. Hierdurch kann vorteilhafte beispielsweise eine Vergleichbarkeit und Transparenz auf dem Markt medizinischer Anwendungen oder Anbieter geschaffen werden. Ebenso können derartige objektive Metriken beispielsweise für Marketingzwecke verwendet werden.

Besonders vorteilhaft kann durch das automatische Erfassen und Abspeichern der genannten Daten beziehungsweise Größen jedoch nicht nur eine beispielsweise statistische Auswertung, sondern ebenso eine Visualisierung der jeweiligen Prozedur oder des jeweiligen Ablaufs, beispielsweise auf einem Dashboard oder in einer Dashboard-Applikation, ermöglicht werden. Dies kann sowohl während der Prozedur in Echtzeit erfolgen als auch nachträglich. Insgesamt können die automatisch erfassten, bestimmten und gespeicherten Daten vorteilhaft beispielsweise für Dokumentation- und/oder Lehrzwecke verwendet werden, wobei eine Visualisierung die Daten auch für Laien besonders einfach und anschaulich verständlich machen beziehungsweise aufbereiten kann.

Besonders vorteilhaft kann eine Zustandsänderung des medizinischen Objekts, insbesondere in Verbindung mit der jeweiligen Position und dem jeweiligen Detektionszeitpunkt, an der beziehungsweise zu dem die Zustandsänderung erfolgt, automatisch detektiert und in der Datenbank gespeichert werden. Eine solche Zustandsänderung kann beispielsweise ein Aufblasen (Ballooning) des Katheters, ein Entfalten einer Gefäßstütze oder dergleichen sein. Hierdurch kann vorteilhaft ein noch besonders detailliertes Nachvollziehen oder Analysieren der jeweiligen Prozedur ermöglicht werden.

In vorteilhafter Ausgestaltung der vorliegenden Erfindung werden das medizinische Objekt und dessen Position durch Erkennen wenigstens eines an dem medizinischen Objekt angeordneten Markers detektiert und nachverfolgt. Mit anderen Worten kann das Nachverfolgen der Position des medizinischen Objekts durch den Marker, beispielsweise eine Markierung oder einen Positionsgeber, an dem medizinischen Objekt selbst und dessen Detektion durch die Bildauswertung beziehungsweise Bildverarbeitung der Bilddaten erfolgen. Eine Art des Markers kann dabei abhängig sein von dem verwendeten bildgebenden Verfahren, sodass jeweils sichergestellt ist, dass der Marker mittels des jeweiligen bildgebenden Verfahrens erfasst beziehungsweise abgebildet, also dargestellt werden kann. Ein solcher Marker kann vorteilhaft eine besonders präzise und zuverlässige Detektion und Lokalisierung des medizinischen Objekts beziehungsweise von dessen Position ermöglichen. Bei einer entsprechenden Ausgestaltung oder Anordnung des Markers kann zudem automatisch und eindeutig eine Ausrichtung oder räumliche Lage des medizinischen Objekts bestimmt werden. Da der Marker an einer bekannten Stelle des medizinischen Objekts angeordnet sein kann, können durch die besonders einfach, zuverlässig und präzise mögliche Detektion des Markers beispielsweise auch jeweilige Ränder oder Außengrenzen des medizinischen Objekts besonders präzise und zuverlässig lokalisiert werden. Durch die Verwendung des Markers kann das erfindungsgemäße Verfahren also insgesamt zuverlässiger und mit verbesserter Präzision oder Genauigkeit durchgeführt werden.

Zusätzlich oder alternativ können manuelle An- oder Eingaben zum Nachverfolgen der Position des medizinischen Objekts erfasst und verarbeitet oder ausgewertet werden. So kann beispielsweise das jeweilige medizinische Personal manuell an- oder eingeben, dass das medizinische Objekt eine bestimmte Position oder Stellung oder einen bestimmten Zustand erreicht hat. Hierdurch kann beispielsweise die automatische Bildauswertung zum Nachverfolgen der Position des medizinischen Objekts unterstützt beziehungsweise abgesichert werden, in dem verlässliche Referenzpunkte geschaffen werden. Somit wird eine Plausibilisierung der automatischen Detektion, Positionsbestimmung und nach Verfolgung des medizinischen Objekts ermöglicht.

In vorteilhafter Ausgestaltung der vorliegenden Erfindung wird das medizinische Objekt vor dem Erfassen der Bilddaten detektiert, insbesondere durch Auslesen eines an dem medizinischen Objekt angeordneten QR-Codes und/oder RFID-Transponders. Ein jeweiliger entsprechender Detektionsort und Detektionszeitpunkt werden dann in der Datenbank gespeichert.

Mit anderen Worten kann so das medizinische Objekt also bereits vor einem Kontakt mit dem Patienten automatisch erfasst beziehungsweise nachverfolgt werden. Hierdurch kann vorteilhaft die gesamte Prozedur einschließlich einer Vorbereitungsphase lückenlos nachvollziehbar und analysierbar gemacht werden. Besonders bevorzugt kann es vorgesehen sein, dass das medizinische Objekt, sofern es nicht bestimmungsgemäß in dem Patienten verbleibt, nach seinem Entfernen aus dem Patienten in gleicher Art und Weise nochmals erfasst beziehungsweise detektiert wird. So kann vorteilhaft automatisch eindeutig, zuverlässig und objektiv dokumentiert werden, welche medizinischen Objekte sich zu welchem Zeitpunkt in dem Patienten oder außerhalb des Patienten befinden. Hierdurch können entsprechende Fehler besonders zuverlässig, insbesondere auch automatisch, erkannt werden. So können beispielsweise eine automatische Überwachung oder ein automatischer Abgleich mit einem vorgegebenen geplanten Prozedurablauf (Workflow) durchgeführt werden, in dem für jedes verwendete medizinische Objekt vermerkt ist, ob es zum Verbleib in dem Patienten vorgesehen ist oder nicht.

Insbesondere für kleinere medizinische Objekte, an denen kein QR-Code und/oder RFID-Transponder angeordnet werden kann, kann eine automatische bildliche Erfassung, beispielsweise mittels einer 3D Kamera vorgesehen sein. Dabei können von der 3D Kamera erzeugte und bereitgestellte Kameradaten analog zu den Bilddaten verarbeitet beziehungsweise ausgewertet werden, um das jeweilige medizinische Objekt zu detektieren und/oder nachzuverfolgen.

In weiterer vorteilhafter Ausgestaltung der vorliegenden Erfindung wird die jeweils aktuelle detektierte Position des medizinischen Objekts, also dessen zuletzt bestimmte Position, mit der Zielposition verglichen. Ein jeweils aktueller Abstand des medizinischen Objekts zu der Zielposition wird dann mittels einer Ampelanzeige visualisiert, wobei bei einem Unter- oder Überschreiten wenigstens eines vorgegebenen Abstandsschwellenwertes die Ampelanzeige automatisch in einen anderen Zustand geschaltet wird. Mit anderen Worten können also beispielsweise wenigstens zwei, bevorzugt wenigstens drei, Werteintervalle für den Abstand beziehungsweise einen Betrag des Abstandes vorgegeben werden. Je nachdem in welches dieser Werteintervallen der jeweils aktuelle Abstand beziehungsweise dessen Wert fällt, ist die Ampelanzeige dann in einen entsprechenden, dem jeweiligen Werteintervall zugeordneten Zustand beziehungsweise Anzeigezustand geschaltet. Die unterschiedlichen Zustände der Ampelanzeige können sich bevorzugt zumindest durch unterschiedliche Farben unterscheiden.

Vergleichbar mit einer herkömmlichen aus dem Verkehrsgeschehen bekannten Lichtsignalanlage (Ampel) kann die vorliegend vorgesehene Ampelanzeige beispielsweise einen rot leuchtenden Zustand, einen gelb leuchtenden Zustand und einen grün leuchtenden Zustand aufweisen. Wird beispielsweise als das medizinische Objekt ein Katheter erkannt, der in Richtung einer Stenose bewegt wird, so kann die Ampelanzeige beispielsweise durch ein rotes Leuchten signalisieren oder visualisieren, dass sich der Katheter noch mehr als 5 mm von der Stenose beziehungsweise der entsprechenden Zielposition, beispielsweise einem Mittel- oder Endpunkt der Stenose, entfernt befindet. Nähert sich der Katheter bei weiterer Annäherung an die Stenose dieser bis auf weniger als beispielsweise 5 mm an, so kann die Ampelanzeige dies durch ein Umschalten auf ein gelbes Leuchten signalisieren. In diesem Beispiel ist ein erster Abstandsschwellenwert also als 5 mm vorgegeben. Als zweiter Abstandsschwellenwert kann beispielsweise eine Entfernung von 2 mm vorgegeben sein. Nähert sich dann der Katheter auf weniger als 2 mm an die Stenose beziehungsweise die entsprechenden Zielposition an, so kann die Ampelanzeige dies durch ein Umschalten auf ein grünes Leuchten visualisieren. Dabei können der beziehungsweise die Abstandsschwellenwerte je nach Bedarf, Anwendung oder Situation unterschiedlich angepasst oder vorgegeben werden.

Eine derartige intervall- oder abstandsschwellenwertbasierte Ampelanzeige kann vorteilhaft eine besonders schnelle und eindeutige Erkennbarkeit einer jeweiligen Situation ermöglichen. Um die jeweilige Farbe der Ampelanzeige wahrzunehmen beziehungsweise zu erkennen, muss das jeweilige medizinische Personal beispielsweise ihre visuelle Aufmerksamkeit nicht oder nur zu einem minimalen Anteil auf die Ampelanzeige lenken, da Farben bereits ohne Fokussierung in einem peripheren Sichtbereich zuverlässig wahrgenommen und identifiziert werden können. Weiterhin ist keine zeit- und konzentrationsaufwendige Interpretation der dargestellten Bilddaten, beispielsweise also von Röntgenbildern, durch das jeweilige medizinische Personal oder andere Personen notwendig, die daher beispielsweise zu Lehrzwecken oder als Laie die jeweilige Prozedur besonders leicht nachvollziehen können.

Besonders vorteilhaft kann ebenso eine komplexere Farbkodierung und/oder beispielsweise eine bestimmte Anordnung oder Reihenfolge der farblich unterschiedlichen Leuchtsignale beziehungsweise Signalquellen, beispielsweise Lampen oder Leuchtdioden, vorgesehen sein. Hierdurch kann vorteilhaft beispielsweise visualisiert werden, auf welcher Seite der Zielposition sich das medizinische Objekt befindet und/oder ob es sich von der Zielposition entfernt oder an die Zielposition annähert. So kann beispielsweise visualisiert werden, dass sich das medizinische Objekt nach einer Annäherungsphase an die Zielposition nunmehr über diese hinaus bewegt hat. Selbstverständlich kann die Ampelanzeige mehr oder weniger als beispielsweise drei unterschiedliche Farben oder Zustände darstellen beziehungsweise aufweisen. So kann beispielsweise ein kontinuierliches Farbband, also beispielsweise ein zusammenhängender Ausschnitt aus einem Farbraum, synchron zu der detektierten Bewegung des medizinischen Objekts, insbesondere in Relation zu der Zielposition, durchlaufen werden. Während also eine auf wenige, beispielsweise auf drei, beschränkte Anzahl von unterschiedlichen Farben oder Zuständen der Ampelanzeige vorteilhaft eine besonders schnelle Erkennbarkeit ermöglichen kann, kann eine größere Anzahl von Farben oder Zuständen der Ampelanzeige eine präzisere Angabe oder Einsetzbarkeit des jeweiligen Abstandes ermöglichen.

Die automatische Bestimmung des jeweiligen Abstandes und dessen besonders einfach erkennbare Anzeige mittels der Ampelanzeige kann auch deshalb besonders vorteilhaft sein, weil eine automatische Bestimmung des Abstandes, insbesondere bei relativ schlechter Bildqualität der Bilddaten und/oder deren Darstellung, präziser sein kann als eine manuelle beziehungsweise rein visuelle Bestimmung oder Einschätzung des Abstandes durch das jeweilige medizinische Personal.

Durch die Ampelanzeige kann die jeweilige Prozedur also schneller und/oder mit verbesserter Genauigkeit und/oder Zuverlässigkeit durchgeführt werden. Weiterhin kann die automatische Bestimmung des Abstandes insbesondere bei relativ schlechter Bildqualität noch zuverlässig und präzise funktionieren, sodass beispielsweise eine dem Patienten verabreichte Kontrastmittelmenge gegenüber herkömmlichen Verfahren reduziert werden kann, bei denen sich beispielsweise der jeweilige behandelnde Arzt allein auf sein Augenmaß und seine korrekte Interpretation der dargestellten Bild- beziehungsweise Röntgendaten verlassen muss. Somit kann durch die Verwendung der Ampelanzeige eine Belastung des Patienten reduziert werden.

In der vorliegenden Erfindung wird das medizinische Objekt, insbesondere dessen Art, Modell und/oder Materialzusammensetzung, identifiziert. Wenigstens ein Parameter eines Visualisierungsalgorithmus, mittels welchem die Bilddaten visualisiert, also beispielsweise auf einem Bildschirm dargestellt, werden, wird dann in Abhängigkeit von der Identifikation des medizinischen Objekts automatisch angepasst, um eine Bildqualität der Visualisierung zu verbessern. Mit anderen Worten kann also in Abhängigkeit von dem abgebildeten medizinischen Objekt beziehungsweise dessen Eigenschaften eine unterschiedliche Bildoptimierung automatisch ausgewählt und durchgeführt werden. Dazu können mehrere Einstellung oder Werte (Presets) für den oder mehrere Parameter vorgegeben werden. Ebenso kann jedoch eine algorithmusbasierte dynamische Anpassung des jeweils aktuellen oder vorherigen Wertes erfolgen, beispielsweise solange bis ein vorgegebenes Kriterium Erfüllt oder erreicht ist. Derartige Kriterien können beispielsweise eine bestimmte Schärfe, ein bestimmtes Signal-Rausch-Verhältnis, ein bestimmter Kontrastwert oder dergleichen sein oder betreffen. Der wenigstens eine angepasste Parameter beziehungsweise die automatische Optimierung kann - wenn es sich bei den Bilddaten beispielsweise um Rohdaten handelt - bereits bei einer Rekonstruierung von Bildern aus diesen Rohdaten verwendet werden. Zusätzlich oder alternativ kann der wenigstens eine angepasste Parameter beziehungsweise die automatische Bildoptimierung bei einer Nachbearbeitung (Postprocessing) von, beispielsweise bereits rekonstruierten, Bildern verwendet werden. Durch eine derartige inhaltsbezogene beziehungsweise inhaltsbasierte Optimierung oder Anpassung der Visualisierung beziehungsweise der Bildqualität kann vorteilhaft eine verbesserte Erkennbarkeit zumindest des medizinischen Objekts erreicht werden.

Ebenso kann beispielsweise eine Datenbank oder eine Zuordnungstabelle bereitgestellt werden, welche unterschiedlichen medizinischen Objekten und/oder Materialzusammensetzungen jeweils einen oder mehrere vorbestimmte optimale beziehungsweise zu verwendende Parameter beziehungsweise Parameterwerte zuordnet. Eine derartige Datenbank oder Tabelle kann dabei nach ihrer Erstellung und Bereitstellung eine besonders einfache, schnelle und rechenaufwandsarme Verbesserung der Bildqualität durch entsprechende Anpassung der Visualisierung beziehungsweise des Visualisierungsalgorithmus ermöglichen.

Es kann dabei besonders vorteilhaft sein, für unterschiedliche Teilbereiche der Bilddaten beziehungsweise für unterschiedliche Teilbereiche eines jeweiligen aus den Bilddaten generierten Bildes die wenigstens einen Parameter unterschiedlich anzupassen. Mit anderen Worten können also für unterschiedliche Teilbereiche des jeweiligen Bildes unterschiedliche Parameterwerte des Visualisierungsalgorithmus verwendet werden, in Abhängigkeit davon, ob in dem jeweiligen Teilbereich das medizinische Objekt sichtbar beziehungsweise abgebildet ist oder nicht. Ebenso können unterschiedliche Teilbereiche des jeweiligen Bildes beispielsweise unterschiedlich optimiert beziehungsweise visualisiert werden, wenn in diesen unterschiedlichen Teilbereichen unterschiedliche medizinische Objekte und/oder beispielsweise unterschiedliche Gewebearten abgebildet sind. So kann insgesamt durch eine inhaltsbasierte Auswertung der Bilddaten die Bild- beziehungsweise Darstellungsqualität verbessert werden.

Zum Identifizieren des medizinischen Objekts, von dessen Art, Modell und/oder Materialzusammensetzung, kann beispielsweise ein automatischer Objekterkennungsalgorithmus verwendet werden. Ebenso kann beispielsweise ein Datenspeicher, wie etwa eine elektronische Patientenakte oder ein elektronisch gespeicherter Plan der Prozedur, von einer entsprechenden Datenverarbeitungseinrichtung automatisch abgefragt werden. Hierdurch können die verwendeten medizinischen Objekte direkt oder, beispielsweise indirekt über die Art der durchgeführten Prozedur, bestimmt werden. Gegebenenfalls kann so aber zumindest eine Menge von möglichen, infrage kommenden medizinischen Objekten eingeschränkt werden, wodurch eine Genauigkeit oder Zuverlässigkeit der Erkennung verbessert werden kann. Zusätzlich oder alternativ kann ebenso beispielsweise eine entsprechende Benutzereingabe erfasst werden, welche das verwendete medizinische Objekt angeben oder identifizieren kann. Weiterhin kann zusätzlich oder alternativ beispielsweise eine Spezifikationsdatenbank automatisch abgefragt werden, um - etwa nach Identifizierung der Art oder des Modells des medizinischen Objekts - weitere Spezifikationen des medizinischen Objekts, wie beispielsweise dessen Materialzusammensetzung, zu bestimmen. Ebenso kann beispielsweise ein Spektrometer verwendet werden oder vorhandene Bilddaten spektrometrisch ausgewertet werden, um beispielsweise die Materialzusammensetzung zu bestimmen. Dann kann auch aus der Materialzusammensetzung ein Rückschluss auf die Art oder das Modell des medizinischen Objekts möglich sein.

Zum Verbessern der Bildqualität in Abhängigkeit von der Identifikation des medizinischen Objekts kann beispielsweise eine Filterung, eine Helligkeit und/oder ein Kontrastverhältnis angepasst werden. Es ist unmittelbar einsichtig, dass beispielsweise metallische medizinische Objekte und medizinische Objekte aus einem Kunststoffmaterial oder aus Glas deutlich unterschiedlich in den Bilddaten repräsentiert sein können. Wird dieser Umstand berücksichtigt, so kann die Bildqualität und die Erkennbarkeit gegenüber herkömmlichen Verfahren, die üblicherweise eine einheitliche, homogenisierte Bilddarstellung oder Bildoptimierung verwenden, deutlich verbessert werden.

In einem Beispiel, dass jedoch nicht Teil der Erfindung ist, und nur zur Veranschaulichung dargestellt wird, kann das medizinische Objekt, insbesondere dessen Art, Modell und/oder Materialzusammensetzung, identifiziert und dann eine für das medizinische bildgebende Verfahren verwendete Strahlungsleistung und/oder Kontrastmittelzuführung beziehungsweise -dosierung in Abhängigkeit von der Identifikation automatisch gesteuert. Ergibt die Identifikation beispielsweise, dass das medizinische Objekt aus einem metallischen Werkstoff gefertigt ist, so kann beispielsweise eine verwendete Strahlungsleistung reduziert werden, da metallische Objekte auch bei reduzierter Strahlungsleistung ausreichend gut erkennbar und/oder detektierbar sind beziehungsweise sein können. Um andererseits beispielsweise einen Kunststoffschlauch ausreichend gut erkennbar zu machen, kann die Strahlungsleistung beispielsweise automatisch erhöht werden. So kann vorteilhaft je nach Bedarf eine maximale oder ausreichende Bildqualität sichergestellt und gleichzeitig eine - zumindest durchschnittliche - Belastung des Patienten minimiert, also auf ein jeweils notwendiges Maß reduziert beziehungsweise beschränkt werden. Das medizinische Objekt kann dabei wie vorliegend bereits an anderer Stelle beschrieben identifiziert werden.

In weiterer vorteilhafter Ausgestaltung der vorliegenden Erfindung wird - wenn als das medizinische Objekt ein vorbestimmtes Objekt detektiert wird und/oder wenn das medizinische Objekt eine vorgegebene Triggerposition erreicht - automatisch ein dies angebendes oder anzeigendes Signal erzeugt und ausgesendet, um einen nachfolgenden Verfahrensschrittes eines medizinischen Verfahrens zu veranlassen oder auszulösen (zu triggern). Beispielsweise bedeutet das Detektieren eines bestimmten medizinischen Objekts, dass dieses sich an dem Detektionsort, also beispielsweise einem Erfassungsbereich eines für das medizinische bildgebende Verfahren, also zum Erzeugen der Bilddaten verwendeten Gerätes befindet. Insbesondere bei relativ langen und/oder mehrschrittigen Prozeduren kann dann beispielsweise ein bestimmter Spezialist für nachfolgende Schritte der Prozedur automatisch benachrichtigt werden, sodass er sich mit optimalem Timing auf seinen Einsatz vorbereiten beziehungsweise sich an einen entsprechenden Einsatzort begeben kann. In einem anderen Beispiel kann dann, wenn das medizinische Objekt die Triggerposition - die der Zielposition entsprechen kann aber nicht muss - erreicht hat automatisch ein Steuersignal an das bildgebende Gerät gesendet werden, um beispielsweise eine mit besonders hoher Strahlungsleistung und/oder Auflösung durchzuführende Dokumentationsaufnahme, die das medizinische Objekt an der erreichten Position abbildet, automatisch ausgelöst werden. Durch ein derartiges Vorgehen kann vorteilhaft der Ablauf der jeweiligen Prozedur teilweise automatisiert und dadurch schneller und/oder mit höherer Zuverlässigkeit oder Effizienz und/oder mit reduziertem Personalaufwand durchgeführt werden.

In einem Beispiel, dass jedoch nicht Teil der Erfindung ist, und nur zur Veranschaulichung dargestellt wird, wird das medizinische Objekt mittels eines Roboters zu der Zielposition bewegt, wobei der Roboter in Abhängigkeit von der detektierten und nachverfolgten Position des medizinischen Objekts gesteuert wird. Mit anderen Worten kann also durch die automatische Auswertung der Bilddaten eine Regelschleife zum automatischen Positionieren des Objekts an der Zielposition mittels des Roboters erzeugt werden. Durch eine derartige Automatisierung kann beispielsweise das medizinische Objekt besonders schnell und/oder präzise, insbesondere zitterfrei, an der Zielposition positioniert werden. Vorteilhaft kann das medizinische Objekt mittels des Roboters auch zitterfrei beliebig lange präzise an der Zielposition gehalten werden. Hierdurch kann vorteilhaft die Präzision bei der Positionierung des medizinischen Objekts verbessert und beispielsweise ein Verletzungsrisiko des Patienten verringert werden. Der Roboter kann beispielsweise ein Industrieroboter oder ein medizinischer Roboter mit einem mehrachsigen Roboterarm beziehungsweise Manipulator sein, wobei der Roboter bevorzugt wenigstens sechs Freiheitsgrade aufweisen kann, um eine flexible Positionierung des medizinischen Objekts zu ermöglichen. So kann beispielsweise ein Katheter mittels des Roboters durch ein Gefäß des Patienten bewegt werden.

In vorteilhafter Ausgestaltung der vorliegenden Erfindung wird durch die Bilddaten wenigstens ein Teilbereich eines Patienten abgebildet und ein EKG-Signal dieses Patienten erfasst. Der Roboter wird dann in Abhängigkeit von dem EKG-Signal zum Ausgleichen einer aus dem EKG-Signal abgeleiteten Herzbewegung und/oder Atembewegung des Patienten gesteuert. Mit anderen Worten wird also aus dem EKG-Signal automatisch eine Bewegung beziehungsweise ein Bewegungsmuster des Patienten bestimmt und der Roboter synchron zu dieser Bewegung oder diesem Bewegungsmuster gesteuert. Dadurch kann beispielsweise das medizinische Objekt trotz der Herzbewegung, insbesondere wenn es sich an dem Herzen befindet, relativ zu dem Herzen oder einem anderen Anatomiemerkmal, beispielsweise einer Gefäßwand, ortsfest, also in relativer Ruhe oder in einer vorgegebenen räumlichen Beziehung verbleiben beziehungsweise gehalten oder geführt werden. Dies kann durch den Roboter besonders präzise und mit hoher Wiederholgenauigkeit erreicht beziehungsweise durchgeführt werden. Durch den Ausgleich der Herzbewegung beziehungsweise der jeweiligen durch die Herzbewegung verursachten lokalen Bewegung kann vorteilhaft ein unerwünschter Kontakt zwischen dem Patienten und dem medizinischen Objekt vermieden und so das Verletzungsrisiko des Patienten minimiert werden. Zugleich kann durch die stabil aufrecht erhaltene räumliche Relation zwischen dem medizinischen Objekt und einem Teil des Patienten, beispielsweise dem Herzen, ein jeweiliger medizinischer Eingriff oder eine Manipulation des Patienten besonders präzise und zuverlässig durchgeführt werden. Es kann dabei vorteilhaft vorgesehen sein, dass der Roboter das medizinische Objekt zwar so bewegt, dass die Herzbewegung ausgeglichen wird, das medizinische Objekt aber dennoch, beispielsweise durch den jeweiligen behandelnden Arzt, bewegbar ist.

Ein erfindungsgemäßes Trackingsystem dient beziehungsweise ist eingerichtet zum automatischen Nachverfolgen eines medizinischen Objekts. Dazu weist das Trackingsystem eine erste Erfassungseinrichtung, eine zweite Erfassungseinrichtung, eine Bildverarbeitungseinrichtung, eine Anzeigeeinrichtung und eine Speichereinrichtung auf. Die erste Erfassungseinrichtung dient beziehungsweise ist eingerichtet zum kontinuierlichen Erfassen von mittels eines medizinischen bildgebenden Verfahrens gewonnenen Bilddaten. Die zweite Erfassungseinrichtung dient beziehungsweise ist eingerichtet zum Erfassen einer vorgegebenen Zielposition für das medizinische Objekt. Die Bildverarbeitungseinrichtung dient beziehungsweise ist eingerichtet zum Detektieren des medizinischen Objekts durch Verarbeitung der Bilddaten mittels eines Bildverarbeitungsalgorithmus und zum zeitaufgelösten Nachverfolgen einer Position des medizinischen Objekts. Die Anzeigeeinrichtung dient beziehungsweise ist eingerichtet zum Anzeigen, dass beziehungsweise wenn das detektierte medizinische Objekt die Zielposition erreicht hat. Die Speichereinrichtung dient beziehungsweise ist eingerichtet zum Abspeichern von mehreren der detektierten Positionen des medizinischen Objekts und zugehöriger Detektionszeitpunkte in einer Datenbank.

Die erste und die zweite Erfassungseinrichtung können separate Einrichtungen oder in einer einzigen Einrichtung vereint sein.

Insbesondere kann das erfindungsgemäße Trackingsystem zum Durchführen zumindest einer Ausführungsform des erfindungsgemäßen Verfahrens ausgebildet und eingerichtet sein. Dazu kann das Trackingsystem beispielsweise ein Speichermedium oder einen Datenträger aufweisen mit einem Programmcode, der die Verfahrensschritte des erfindungsgemäßen Verfahrens repräsentiert beziehungsweise kodiert. Das Trackingsystem kann weiterhin wenigstens eine Mikrochip- oder Mikroprozessoreinrichtung aufweisen, welche zum Ausführen dieses Programmcodes eingerichtet ist. Das Trackingsystem kann zudem eine oder mehrere Schnittstellen und/oder wenigstens ein Benutzerinterface aufweisen, worüber Daten und/oder Eingaben empfangen beziehungsweise erfasst werden können. Ebenso kann wenigstens eine Ausgabeschnittstelle vorgesehen sein, um beispielsweise Daten oder eine Visualisierung an ein externes System oder eine externe Datenbank auszugeben.

Die bisher und im Folgenden angegebenen Eigenschaften und Weiterbildungen des erfindungsgemäßen Verfahrens sowie die entsprechenden Vorteile sind jeweils sinngemäß auf das erfindungsgemäße Trackingsystem und/oder zur Durchführung des erfindungsgemäßen Verfahrens verwendete oder verwendbare Bauteile und Einrichtungen übertragbar und umgekehrt. Es gehören also zu der Erfindung auch solche Weiterbildungen des erfindungsgemäßen Verfahrens und des erfindungsgemäßen Trackingsystems, welche Ausgestaltungen aufweisen, die hier nicht explizit in der jeweiligen Kombination beschrieben sind.

Weitere Merkmale, Einzelheiten und Vorteile der vorliegenden Erfindung ergeben sich aus der nachfolgenden Beschreibung bevorzugter Ausführungsbeispiele sowie anhand der Zeichnungen. Dabei zeigen:
- FIG 1: eine schematisch und ausschnittweise Veranschaulichung von medizinischen Bilddaten und eines Trackingsystems zum Nachverfolgen eines medizinischen Objekts zu einem ersten Zeitpunkt;
- FIG 2: schematisch die Veranschaulichung aus FIG 1 zu einem späteren zweiten Zeitpunkt; und
- FIG 3: einen schematischen beispielhaften Ablaufplan eines Verfahrens zum Nachverfolgen eines medizinischen Objekts.

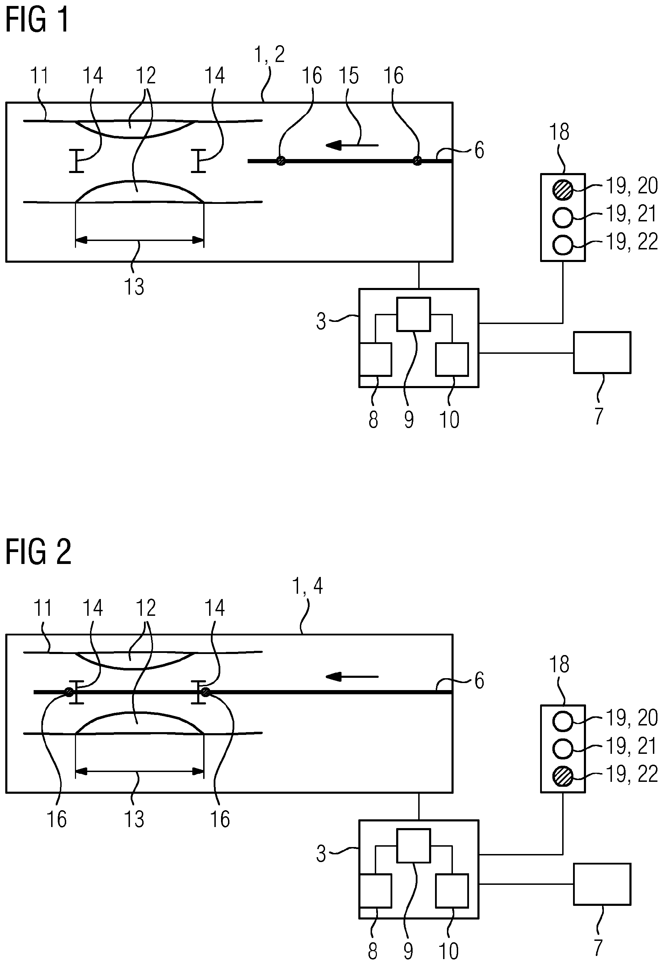

FIG 1 zeigt eine schematische und ausschnittsweise Veranschaulichung von medizinischen Bilddaten 1 zu einem ersten Zeitpunkt 2 und ein Trackingsystem 3 zum Nachverfolgen eines medizinischen Objekts.

FIG 2 zeigt die schematische Veranschaulichung aus FIG 1 zu einem späteren, zweiten Zeitpunkt 4.

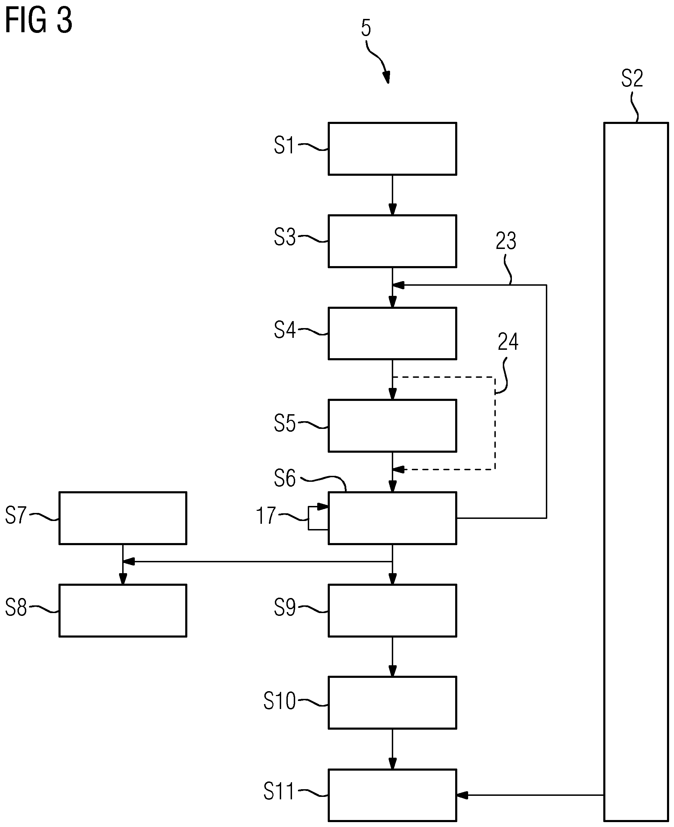

FIG 3 zeigt einen schematischen beispielhaften Ablaufplan 5 eines Verfahrens zum Nachverfolgen des medizinischen Objekts mittels des Trackingsystems 3. Im Folgenden werden die Verfahrensschritte dieses Verfahrens unter Bezugnahme auf FIG 1, FIG 2 und FIG 3 erläutert.

In einem Verfahrensschritt S1 wird das Verfahren gestartet. Hier kann beispielsweise das Trackingsystem 3 in Betrieb genommen werden, es können für die weiteren Verfahrensschritte benötigte oder nützliche Daten bereitgestellt beziehungsweise eine entsprechende Datenbank für das Trackingsystem 3 zugänglich gemacht und ähnliche vorbereitende Maßnahmen durchgeführt werden.

In einem parallel zu den weiteren Verfahrensschritten durchgeführten Verfahrensschritts S2 werden von dem Trackingsystem 3 erfasste und gegebenenfalls erzeugte sowie gegebenenfalls dem Trackingsystem 3 bereitgestellte Daten visualisiert. So können beispielsweise ein jeweiliger Ort und/oder Status eines jeweiligen durch die medizinischen Bilddaten 1 abgebildeten Patienten und/oder des medizinischen Objekts angezeigt werden. Ebenso können beispielsweise jeweilige Schritte einer den Patienten betreffenden Prozedur, ein Belegungsstatus jeweiliger hierfür verwendeter Räumlichkeiten, ein Zeitstrahl mit während der Prozedur auftretenden Ereignissen und/oder dergleichen mehr visualisiert, also beispielsweise auf einem Bildschirm beziehungsweise einem medizinischen Dashboard angezeigt werden. Diese Visualisierung kann beispielsweise farbcodiert und/oder anderweitig grafisch aufbereitet sein, um sämtliche für die Prozedur relevanten Daten gebündelt verfügbar und nachvollziehbar zu machen.

Bei dem medizinischen Objekt kann es sich im vorliegenden Beispiel etwa um einen Katheter 6 handeln. Der Katheter 6 kann beispielsweise einen QR-Code oder einen RFID-Transponder oder -Tag aufweisen, durch den der Katheter 6 als solcher identifiziert wird. Zusätzlich oder alternativ können beispielsweise in im QR-Code beziehungsweise in dem RFID-Tag eine Identifikationsnummer, eine Modellnummer und/oder weitere Daten, beispielsweise betreffend eine Spezifikation des Katheters 6, gespeichert beziehungsweise kodiert sein.

In einem Verfahrensschritt S3 kann der Katheter 6 beispielsweise in einen Operationssaal, in dem der Patient als Teil der Prozedur einem Eingriff oder einer Behandlung unterzogen wird, gelangen beziehungsweise verbracht werden. Dabei kann der QR-Code beziehungsweise der RFID-Tag automatisch von dem Trackingsystem 3 ausgelesen werden. Das Trackingsystem 3 kann dann beispielsweise automatisch auf eine Datenquelle 7 zugreifen, um etwa anhand der Identifikationsnummer des Katheters 7 weitere Daten oder Spezifikationen bezüglich des Katheters 7 abzurufen. Somit können also bereits zu diesem Zeitpunkt, also vor Beginn des eigentlichen Eingriffs an dem Patienten, der Standort des Katheters 6 in dem Operationssaal und gegebenenfalls weitere Eigenschaften des Katheters 6 Trackingsystem 3 bekannt sein und dementsprechend auch visualisiert werden. Damit ist für jede Person, die Zugriff auf die Visualisierung beziehungsweise die entsprechenden der Visualisierung zugrundeliegenden Daten hat, sofort ersichtlich, wo sich welches medizinische Objekt zu welchem Zeitpunkt befindet. Hierdurch kann nicht nur eine Logistik vereinfacht und/oder überprüfbar gemacht werden, sondern es kann bereits vor Beginn des eigentlichen Eingriffs an dem Patienten sichergestellt werden, dass sämtliches für die Prozedur benötigtes medizinisches Gerät und Material tatsächlich in dem Operationssaal verfügbar ist.

Auch kann hier beispielsweise der Operationssaal automatisch als belegt gekennzeichnet beziehungsweise in der Visualisierung angezeigt werden, beispielsweise wenn der Patient in dem Operationssaal eintrifft. Dies kann beispielsweise durch automatisches Scannen, also Auslesen eines an einer Patientenliege angeordneten QR-Codes von dem Trackingsystem 3 erfasst werden.

Zum Erfassen dieser und weiterer Daten kann das Trackingsystem beispielsweise eine Erfassungseinrichtung 8 aufweisen. Die erfassten Daten können von einer Datenverarbeitungseinrichtung 9 des Trackingsystems 3 verarbeitet werden. Zudem kann das Trackingsystem 3 eine Speichereinrichtung 10 zum Speichern der Daten und/oder sprechender Verarbeitungsergebnisse, also von der Datenverarbeitungseinrichtung 9 ausgegebener Daten, aufweisen. Je nach Funktionalität oder Funktionsumfang kann das Trackingsystem auch noch weitere Einrichtungen oder Bauteile aufweisen.

Der Katheter 6 dient lediglich als Beispiel für das medizinische Objekt, da selbstverständlich ebenso weitere oder andere medizinische Objekte, Geräte, Materialien und dergleichen entsprechend erfasst werden können.

In einem Verfahrensschritt S4 können die Bilddaten 1 erfasst werden. Dazu kann der Patient beispielsweise mittels eines Röntgengeräts oder eines Angiographiesystems abgebildet werden. Die Bilddaten 1 werden also mittels eines medizinischen bildgebenden Verfahrens erfasst. Zusätzlich zu einer herkömmlichen Darstellung der Bilddaten 1 auf einem Bildschirm oder einer Anzeigefläche können sie dem Trackingsystem 3 bereitgestellt beziehungsweise von diesem verarbeitet werden.

Vorliegend können die Bilddaten 1 beispielsweise ein Gefäß 11 des Patienten mit einer Stenose 12 einer definierten Länge 13 abbilden. In einem Verfahrensschritt S5 kann von dem Trackingsystem 3 eine Zielposition für den Katheter 6 erfasst werden. Dazu können beispielsweise von einem den Patienten behandelnden Arzt eine Idealposition, also die Zielposition, anzeigende Zielmarkierungen 14 an einem Anfang und einem Ende der Stenose 12 vorgegeben werden. Ebenso kann es möglich sein, durch automatische Bildverarbeitung der Bilddaten 1 die Länge 13 der Stenose 12 und/oder beispielsweise einen Mittelpunkt der Stenose 12 automatisch zu bestimmen.

Während des Eingriffs führt nun der Arzt den Katheter 6 in den Patienten, insbesondere in das Gefäß 11, ein. Dementsprechend wird der Katheter 6 ebenso wie das Gefäß 11 mittels des bildgebenden Verfahrens erfasst und ist daher ebenfalls in den Bilddaten 1 abgebildet. Eine Bewegung des Katheters 6 in Richtung der Stenose 12 ist hier durch einen entsprechenden Pfeil 15 angedeutet.

In einem Verfahrensschritt S6 werden die Bilddaten 1 von dem Trackingsystem 3 verarbeitet, beispielsweise mittels eines Objekterkennungsalgorithmus. Dabei kann das Trackingsystem automatisch den Katheter 6 detektieren und seine Position, insbesondere relativ zu der Zielposition beziehungsweise der Stenose 12 bestimmen. Hierzu kann der Katheter 6 beispielsweise röntgensichtbare Marker 16 aufweisen, durch die das Detektieren des Katheters 6 und/oder das Bestimmen seiner Position vereinfacht werden können. Das Trackingsystem 3 kann dabei nicht nur automatisch erkennen, dass in den Bilddaten 1 ein medizinisches Objekt abgebildet ist, sondern dieses auch als den Katheter 6 identifizieren. Dazu kann es beispielsweise vorab bereitgestellte und/oder aus der Datenquelle 7 und/oder aus der Speichereinrichtung 10 abgerufene Daten bearbeiten beziehungsweise berücksichtigen. Das Trackingsystem 3 kann also erkennen beziehungsweise bestimmen, was in den Bilddaten 1 dargestellt ist. Da das Trackingsystem 3 die kontinuierlich aktualisierten Bilddaten 1 ebenso kontinuierlich auswerten kann, kann das Trackingsystem die jeweils aktuelle Position des Katheters 6 kontinuierlich nachverfolgen, was in dem Ablaufplan 5 durch eine erste Schleife 17 angedeutet ist.

Insbesondere kann das Trackingsystem 3 einen Abstand von dem Katheter 6 in seiner jeweils aktuellen Position zu der Zielposition bestimmen und kontinuierlich aktualisieren. Dieser Abstand kann je nach Bedarf auf unterschiedliche Weisen bestimmt beziehungsweise definiert werden. So kann als der Abstand beispielsweise eine Entfernung zwischen einer Spitze des Katheters 6 und einem Mittelpunkt der Stenose 12 bestimmt sein. Der Mittelpunkt der Stenose 12 kann dabei beispielsweise durch Addieren der Hälfte der Länge 13 zu einer Position einer der Zielmarkierungen 14 in Richtung der anderen Zielmarkierung 14 berechnet werden. Ebenso kann der Abstand beispielsweise als die Entfernung bestimmt werden, die der Katheter 6 noch in Richtung der Stenose 12 beziehungsweise der Zielposition zurücklegen muss, um die Marker 16 in Überdeckung mit den Zielmarkierungen 14 oder in eine zu den Zielmarkierungen 14 symmetrische Lage zu bringen.

In Abhängigkeit von dem jeweils aktuell bestimmten Abstand des Katheters 6 zu der Zielposition kann das Trackingsystem 2 eine Anzeigeeinrichtung ansteuern, die vorliegend als Ampelanzeige 18 ausgebildet ist. Diese Ampelanzeige 18 weist vorliegend beispielhaft drei Anzeigeelemente 19 auf. Von diesen kann beispielsweise ein lerstes Anzeigeelement 20 in einem rot leuchtenden Zustand, ein zweites Anzeigeelement 21 in einen gelb leuchtenden Zustand und ein drittes Anzeigeelement 22 in einen grün leuchtenden Zustand geschaltet werden. Vorliegend beträgt zum in FIG 1 dargestellten ersten Zeitpunkt 2 der Abstand mehr als beispielsweise 5 mm oder beispielsweise mehr als 10 mm. Daher ist die Ampelanzeige 18 zum ersten Zeitpunkt 2 in einen Zustand geschaltet, in dem die Ampelanzeige 18 durch ein rotes Leuchten des ersten Anzeigeelements 20 diesen Abstandswert anzeigt.

Beispielsweise in Abhängigkeit von einer von dem Trackingsystem 3 bestimmten Identifikation des Katheters 6 und/oder in Abhängigkeit von dem bestimmten Abstand des Katheters 6 zu der Zielposition kann automatisch eine zum Erzeugen der Bilddaten 1 verwendete Strahlungsleistung und/oder Kontrastmittelzuführung angepasst werden. Ebenso kann beispielsweise in Abhängigkeit von der bestimmten Identifikation des Katheters 6 ein zum Rekonstruieren und/oder Anzeigen der Bilddaten 1 verwendeter Visualisierungsalgorithmus angepasst werden, um eine optimale Bildqualität zu erzielen. Diese Anpassungen sind im Ablaufplan 5 durch eine zweite Schleife 23 angedeutet.

Nach dem Anpassen der Strahlungsleistung, der Kontrastmittelzuführung und/oder des Visualisierungsalgorithmus können weitere Bilddaten erzeugt beziehungsweise erfasst und/oder visualisiert werden. Es ist dann gegebenenfalls nicht notwendig die vorgegebene Zielposition erneut zu erfassen oder zu ermitteln. Es kann also möglich sein, dass der Verfahrensschritt S5 während der Prozedur beziehungsweise während des Verfahrens nur einmalig oder nur einmalig für jedes medizinische Objekt durchgeführt wird, was in dem Ablaufplan 5 durch einen Pfad 24 angedeutet ist.

Parallel zu einem, einigen oder allen der bisherigen und/oder der nachfolgenden Verfahrensschritte kann in einem Verfahrensschritt S7 beispielsweise ein EKG-Signal des Patienten erfasst werden.

In Abhängigkeit von diesem EKG-Signal beziehungsweise einer daraus abgeleiteten Herz- und/oder Atembewegung des Patienten und/oder in Abhängigkeit von der bestimmten Identifikation des Katheters 6 und/oder in Abhängigkeit von den Bilddaten 1 beziehungsweise daraus erzeugten Datenverarbeitungsergebnissen, wie beispielsweise der Position des Katheters 6 beziehungsweise dessen relativer Position oder Lage zu der Zielposition, kann in einem Verfahrensschritt S8 ein Roboter gesteuert beziehungsweise angesteuert werden. Dieser Roboter kann im vorliegenden Beispiel also mit dem Trackingsystem 3 gekoppelt sein. Mittels des Roboters 3 kann der Katheter 6 gehalten und insbesondere in die oder zu der Zielposition bewegt werden.

Unterschreitet während dieser Bewegung der Abstand des Katheters 6 zu der Zielposition den vorgegebenen Abstandsschwellenwert von beispielsweise 5 mm oder 10 mm, so wird dies automatisch durch ein gelbes Leuchten des zweiten Anzeigeelements 21 angezeigt. Das rote Leuchten des ersten Anzeigeelements 20 kann dann aufgehoben beziehungsweise ausgeschaltet werden.

Zu dem in FIG 2 schematisch dargestellten zweiten Zeitpunkt 4 hat vorliegend der Katheter 6 die Zielposition erreicht. Dementsprechend wurde der Katheter 6 so weit in Richtung der durch die Zielmarkierungen 14 angezeigten beziehungsweise vorgegebenen Zielposition bewegt, dass die Positionen der Marker 16 des Katheters 6 bestmöglich mit den Zielmarkierungen 14 in Überdeckung beziehungsweise in eine symmetrische Lage oder Anordnung gebracht sind. Dieses Erreichen der Zielposition wird von dem Trackingsystem 3 automatisch durch Ansteuern der Ampelanzeige 18 beziehungsweise durch Umschalten auf ein grünes Leuchten des dritten Anzeigeelements 22 angezeigt. Da also die relative Lage des Katheters 6 zu der Stenose 12 beziehungsweise zu der Zielposition mittels der Ampelanzeige 18 besonders einfach erkennbar angezeigt wird, kann die Ampelanzeige 18 beziehungsweise das Trackingsystem 3 also anschaulich als Navigations- oder Einparkhilfe für den jeweiligen behandelnden Arzt dienen beziehungsweise verstanden werden, insbesondere wenn der Katheter 6 von dem Arzt selbst manuell oder durch Bedienung des Roboters bewegt wird.

Die Zielposition oder eine andere vorgegebene Position kann ebenso als eine Triggerposition definiert sein, bei deren Erreichen durch den Katheter 6 automatisch ein entsprechendes Signal von dem Trackingsystem 3 erzeugt und in einem Verfahrensschritt S10 ausgegeben wird. Dieses Signal kann dann einen weiteren Schritt der Prozedur veranlassen oder auslösen.

Nach Abschluss des Eingriffs an dem Patienten kann durch das Nachverfolgen der verwendeten medizinischen Instrumente, wie also hier beispielsweise des Katheters 6, automatisch und objektiv zuverlässig sichergestellt werden, dass keine medizinischen Objekte unbeabsichtigt in dem Patienten verbleiben. Dazu kann beispielsweise erneut außerhalb des Patienten ein jeweiliger QR-Code oder RFID-Tag ausgelesen werden.

In einem Verfahrensschritt S11 können die während des Verfahrens beziehungsweise während der Prozedur von dem Trackingsystem 3 erfassten, verarbeiteten, erzeugten und/oder visualisierten Daten gespeichert werden, beispielsweise in der Speichereinrichtung 10. Selbstverständlich können die entsprechenden Daten bereits während der vorherigen Verfahrensschritte unmittelbar bei oder nach ihrer jeweiligen Verfügbarkeit gespeichert werden. Diese gespeicherten Daten können dann als Grundlage zum Visualisieren der Prozedur beziehungsweise eines entsprechenden Workflows dienen und/oder beispielsweise eine Analyse auch zu einem späteren Zeitpunkt ermöglichen.

Insgesamt ist es gegenüber bisherigen Methoden also besonders vorteilhaft, die Bilddaten 1 nicht nur während der Prozedur anzuzeigen, sondern eine automatische Auswertung, Verarbeitung und Speicherung vorzunehmen und dazu die entsprechenden Daten an das Trackingsystem 3 weiterzuleiten.

## Patentansprüche

1. Verfahren (5) zum Nachverfolgen eines medizinischen Objekts (6), mit den Verfahrensschritten
- kontinuierliches Erfassen von mittels eines medizinischen bildgebenden Verfahrens gewonnenen Bilddaten (1),
- Erfassen einer vorgegebenen Zielposition (14) für das medizinische Objekt (6),
- Anzeigen, dass das detektierte medizinische Objekt (6) die Zielposition (14) erreicht hat,
**gekennzeichnet durch**
- automatisches Detektieren des medizinischen Objekts (6) und zeitaufgelöstes Nachverfolgen von dessen jeweiliger Position anhand der Bilddaten (1) mittels eines Bildverarbeitungsalgorithmus,
- Ermitteln, dass das medizinische Objekt (6) die Zielposition (14) erreicht hat mittels des Bildverarbeitungsalgorithmus,
- Identifizieren des medizinischen Objekts (6),
- Visualisieren der Bilddaten (1) mittels eines Visualisierungsalgorithmus, wobei wenigstens ein Parameter des Visualisierungsalgorithmus in Abhängigkeit von der Identifikation des medizinischen Objekts (6) automatisch angepasst wird, um eine Bildqualität der Visualisierung zu verbessern, und
- Speichern mehrerer der detektierten Positionen des medizinischen Objekts (6) und zugehöriger Detektionszeitpunkte in einer Datenbank (10).

2. Verfahren (5) nach Anspruch 1, **dadurch gekennzeichnet,**
**dass** das medizinische Objekt (6) und dessen Position durch Erkennen wenigstens eines an dem medizinischen Objekt (6) angeordneten Markers (16) detektiert und nachverfolgt werden.

3. Verfahren (5) nach einem der vorhergehenden Ansprüche, **dadurch gekennzeichnet, dass** das medizinische Objekt (6) vor dem Erfassen der Bilddaten (1), insbesondere durch Auslesen eines an dem medizinischen Objekt (6) angeordneten QR-Codes und/oder RFID-Transponders, detektiert wird und ein jeweiliger entsprechender Detektionsort und Detektionszeitpunkt in der Datenbank (10) gespeichert werden.

4. Verfahren (5) nach einem der vorhergehenden Ansprüche, **dadurch gekennzeichnet, dass**
- die jeweils aktuelle detektierte Position des medizinischen Objekts (6) mit der Zielposition (14) verglichen wird, und
- ein jeweils aktueller Abstand des medizinischen Objekts (6) zu der Zielposition (14) mittels einer Ampelanzeige (18) visualisiert wird, wobei bei einem Unter- oder Überschreiten wenigstens eines vorgegebenen Abstandsschwellenwertes die Ampelanzeige (18) automatisch in einen anderen Zustand (20, 21, 22) geschaltet wird.

5. Verfahren (5) nach einem der vorhergehenden Ansprüche, **dadurch gekennzeichnet, dass**
- zum Identifizieren des medizinischen Objekts (6) dessen Art, Modell und/oder Materialzusammensetzung identifiziert wird.

6. Verfahren (5) nach einem der vorhergehenden Ansprüche, **dadurch gekennzeichnet, dass**
- das medizinische Objekt (6), insbesondere dessen Art, Modell und/oder Materialzusammensetzung, identifiziert wird, und
- eine für das medizinische bildgebende Verfahren verwendete Strahlungsleistung und/oder Kontrastmittelzuführung in Abhängigkeit von der Identifikation automatisch gesteuert wird.

7. Verfahren (5) nach einem der vorhergehenden Ansprüche, **dadurch gekennzeichnet, dass** wenn als das medizinische Objekt (6) ein vorbestimmtes Objekt (6) detektiert wird und/oder wenn das medizinische Objekt (6) eine vorgegebene Triggerposition erreicht automatisch ein dies angebendes Signal erzeugt und ausgesendet wird zum Veranlassen eines nachfolgenden Verfahrensschrittes eines medizinischen Verfahrens.

8. Verfahren (5) nach einem der vorhergehenden Ansprüche, **dadurch gekennzeichnet, dass** das medizinische Objekt (6) mittels eines Roboters zu der Zielposition (14) bewegt wird, wobei der Roboter in Abhängigkeit von der detektierten und nachverfolgten Position des medizinischen Objekts (6) gesteuert wird.

9. Verfahren (5) nach Anspruch 8, **dadurch gekennzeichnet,**
**dass**
- durch die Bilddaten (1) wenigstens ein Teilbereich (11, 12) eines Patienten abgebildet wird,
- ein EKG-Signal des Patienten erfasst wird, und
- der Roboter in Abhängigkeit von dem EKG-Signal zum Ausgleichen einer aus dem EKG-Signal abgeleiteten Herzbewegung des Patienten gesteuert wird.

10. Trackingsystem (3) zum automatischen Nachverfolgen eines medizinischen Objekts (6), aufweisend
- eine erste Erfassungseinrichtung (3, 8) zum kontinuierlichen Erfassen von mittels eines medizinischen bildgebenden Verfahrens gewonnenen Bilddaten (1),
- eine zweite Erfassungseinrichtung (3, 8) zum Erfassen einer vorgegebenen Zielposition (14) für das medizinische Objekt (6),
- eine Anzeigeeinrichtung (18) zum Anzeigen, dass das detektierte medizinische Objekt (6) die Zielposition (14) erreicht hat, und
- eine Speichereinrichtung (10) zum Abspeichern von mehreren der detektierten Positionen des medizinischen Objekts (6) und zugehöriger Detektionszeitpunkte in einer Datenbank (10),
**gekennzeichnet durch**
- eine Bildverarbeitungseinrichtung die dazu eingerichtet ist das medizinische Objekt (6) durch Verarbeitung der Bilddaten (1) mittels eines Bildverarbeitungsalgorithmus automatisch zu detektieren und eine Position des medizinischen Objekts (6) zeitaufgelöst nachzuverfolgen; und wobei der Bildverarbeitungsalgorithmus ausgeführt ist zu ermitteln, dass das medizinische Objekt (6) die Zielposition (14) erreicht hat;
- einen Bildschirm zum Visualisieren der Bilddaten (1), und
- wobei das Trackingsystem (3) dazu eingerichtet ist, das medizinische Objekt (6) zu identifizieren; wobei
- das Trackingsystem (3) dazu eingerichtet ist, wenigstens einen Parameter eines Visualisierungsalgorithmus zum Visualisieren der Bilddaten (1) in Abhängigkeit von der Identifikation des medizinischen Objekts (6) automatisch anzupassen, um eine Bildqualität der Visualisierung zu verbessern.

## Claims

1. Method (5) for tracking a medical object (6) with the method steps
- continuous acquisition of image data (1) obtained by means of a medical imaging method,
- acquisition of a predetermined target position (14) for the medical object (6),
- indication that the detected medical object (6) has reached the target position (14),
**characterised by**
- automatic detection of the medical object (6) and time-resolved tracking of the respective position thereof using the image data (1) by means of an image processing algorithm,
- ascertaining, by means of the image processing algorithm, that the medical object (6) has reached the target position (14),
- identification of the medical object (6),
- visualisation of the image data (1) by means of a visualisation algorithm, wherein at least one parameter of the visualisation algorithm is automatically adapted as a function of the identification of the medical object (6), in order to improve an image quality of the visualisation, and
- storage of a plurality of the detected positions of the medical object (6) and associated detection times in a database (10).

2. Method (5) according to claim 1, **characterised in that** the medical object (6) and the position thereof are detected and tracked by recognising at least one marker (16) arranged on the medical object (6).

3. Method (5) according to one of the preceding claims, **characterised in that** the medical object (6) is detected prior to the acquisition of the image data (1), in particular by reading a QR code and/or RFID transponder arranged on the medical object (6), and a respective corresponding detection location and detection time are stored in the database (10).

4. Method (5) according to one of the preceding claims, **characterised in that**
- the respective present detected position of the medical object (6) is compared with the target position (14) and
- a respective present distance of the medical object (6) to the target position (14) is visualised by means of a traffic light display (18), wherein, when at least one predetermined distance threshold value is exceeded or fallen below, the traffic light display (18) is automatically switched to another state (20, 21, 22).

5. Method (5) according to one of the preceding claims, **characterised in that**
- in order to identify the medical object (6), the type, model and/or material composition thereof is identified.

6. Method (5) according to one of the preceding claims, **characterised in that**
- the medical object (6), in particular the type, model and/or material composition thereof, is identified and
- a radiation power used for the medical imaging method and/or contrast medium feed is automatically controlled as a function of the identification.

7. Method (5) according to one of the preceding claims, **characterised in that** when a predetermined object (6) is detected as the medical object (6) and/or when the medical object (6) reaches a predetermined trigger position a signal indicating this is automatically generated and emitted in order to initiate a subsequent method step of a medical method.

8. Method (5) according to one of the preceding claims, **characterised in that** the medical object (6) is moved by means of a robot to the target position (14), wherein the robot is controlled as a function of the detected and tracked position of the medical object (6).

9. Method (5) according to claim 8, **characterised in that**
- the image data (1) depicts at least one subregion (11, 12) of a patient,
- an ECG signal of the patient is acquired and
- the robot is controlled as a function of the ECG signal in order to compensate a heart movement of the patient derived from the ECG signal.

10. Tracking system (3) for automatic tracking of a medical object (6) comprising
- a first acquisition apparatus (3, 8) for the continuous acquisition of image data (1) obtained by means of a medical imaging method,
- a second acquisition apparatus (3, 8) for the acquisition of a predetermined target position (14) for the medical object (6),
- a display apparatus (18) for indicating that the detected medical object (6) has reached the target position (14) and
- a storage apparatus (10) for storing a plurality of the detected positions of the medical object (6) and associated detection times in a database (10),
**characterised by**
- an image-processing device, which is configured to automatically detect the medical object (6) by processing the image data (1) by means of an image processing algorithm and to track a position of the medical object (6) in a timeresolved manner; and wherein the image processing algorithm is designed to ascertain that the medical object (6) has reached the target position (14);
- a screen for visualising the image data (1), and
- wherein the tracking system (3) is configured to identify the medical object (6), wherein
- the tracking system (3) is configured to automatically adjust at least one parameter of a visualisation algorithm for visualisation of the image data (1) as a function of the identification of the medical object (6), in order to improve an image quality of the visualisation.

## Revendications

1. Procédé (5) de suivi d'un objet (6) médical, comprenant les stades de procédé
- saisie continuelle de données (1) d'image obtenues au moyen d'un procédé d'imagerie médicale,
- relevé d'une position (14) cible donnée à l'avance de l'objet (6) médical,
- indication que l'objet (6) médical détecté a atteint la position (14) cible,
**caractérisé par**
- détection automatique de l'objet (6) médical et suivi avec résolution temporelle de sa position à l'aide des données (1) d'image au moyen d'un algorithme de traitement d'image,
- détermination que l'objet (6) médical a atteint la position (14) cible au moyen de l'algorithme de traitement d'image,
- identification de l'objet (6) médical,
- visualisation des données (1) d'image au moyen d'un algorithme de visualisation, dans lequel au moins un paramètre de l'algorithme de visualisation est adapté automatiquement en fonction de l'identification de l'objet (6) médical afin d'améliorer une qualité d'image de la visualisation, et
- mise en mémoire de plusieurs des positions détectées de l'objet (6) médical et d'instants de détection allant avec dans une base (10) de données.

2. Procédé (5) suivant la revendication 1, **caractérisé en ce que** l'on détecte et on suit l'objet (6) médical et sa position par reconnaissance d'au moins un repère (16) mis sur l'objet (6) médical.

3. Procédé (5) suivant l'une des revendications précédentes, **caractérisé en ce que** l'on détecte l'objet (6) médical avant la saisie des données (1) d'image, notamment par lecture d'un code QR et/ou d'un transpondeur RFID mis sur l'objet (6) médical et on mémorise un emplacement de détection respectif correspondant et un instant de détection dans la base (10) de données.

4. Procédé (5) suivant l'une des revendications précédentes, **caractérisé en ce que**
- on compare la position détectée respective en cours de l'objet (6) médical à la position (14) cible, et
- on visualise au moyen d'un affichage (18) suspendu la distance respectivement en cours de l'objet (6) médical à la position (14) cible, dans lequel si l'on passe en dessous ou au-dessus d'au moins une valeur de seuil de distance donnée à l'avance, on met l'affichage (18) suspendu automatiquement dans un autre état (20, 21, 22).

5. Procédé (5) suivant l'une des revendications précédentes, **caractérisé en ce que**
- pour l'identification de l'objet (6) médical, on identifie son type, son modèle et/ou sa composition de matériau.

6. Procédé (5) suivant l'une des revendications précédentes, **caractérisé en ce que**
- on identifie l'objet (6) médical, notamment son type, son modèle et/ou sa composition de matériau, et
- on commande automatiquement en fonction de l'identification la puissance de rayonnement et/ou l'administration d'agent de contraste utilisé pour le procédé d'imagerie médicale.

7. Procédé (5) suivant l'une des revendications précédentes, **caractérisé en ce que**, si l'on détecte comme objet (6) médical un objet (6) déterminé à l'avance et/ou si l'objet (6) médical atteint une position de déclenchement donnée à l'avance, il est produit et émis un signal l'indiquant pour provoquer un stade suivant d'un procédé médical.

8. Procédé (5) suivant l'une des revendications précédentes, **caractérisé en ce que** l'on déplace l'objet (6) médical au moyen d'un robot à la position (14) cible, dans lequel on commande le robot en fonction de la position détectée et suivie de l'objet (6) médical.

9. Procédé (5) suivant la revendication 8, **caractérisé en ce que**
- par les données (1) d'image, on reproduit au moins une zone (11, 12) partielle d'un patient,
- on relève un signal EKG du patient, et
- on commande le robot en fonction du signal EKG pour compenser un mouvement cardiaque du patient dérivant du signal EKG.

10. Système (3) de suivi pour suivre automatiquement un objet (6) médical, comportant
- un premier dispositif (3, 8) de saisie pour la saisie continuellement de données (1) d'images obtenues au moyen d'un procédé d'imagerie médicale,
- un deuxième dispositif (3, 8) de relevé pour le relevé d'une position (14) cible donnée à l'avance de l'objet (6) médical,
- un dispositif (18) d'indication pour l'indication que l'objet (6) médical détecté a atteint la position (14) cible, et
- un dispositif (10) de mise en mémoire pour la mise en mémoire de plusieurs des positions détectées de l'objet (6) médical et d'instants de détection allant avec dans une base (10) de donnée,
**caractérisé par**
- un dispositif de traitement d'image, qui est conçu pour détecter automatiquement au moyen d'un algorithme de traitement d'image l'objet (6) médical par traitement des données (1) d'image et pour suivre avec résolution temporelle une position de l'objet (6) médical et dans lequel l'algorithme de traitement d'image est réalisé pour déterminer que l'objet (6) médical a atteint la position (14) cible ;
- un écran de visualisation des données (1) d'image, et
- dans lequel le système (3) de suivi est conçu pour identifier l'objet (6) médical ;
dans lequel
- le système (3) de suivi est conçu pour adapter automatiquement au moins un paramètre d'un algorithme de visualisation pour la visualisation des données (1) d'images en fonction de l'identification de l'objet (6) médical, afin d'améliorer la qualité d'images de la visualisation.
